(19)

Europäisches
Patentamt
European
Patent Office
Office européen
des brevets

(11) **EP 2 058 290 A1**

(12) **EUROPEAN PATENT APPLICATION**
published in accordance with Art. 153(4) EPC

(43) Date of publication:
**13.05.2009 Bulletin 2009/20**

(21) Application number: **07806018.3**

(22) Date of filing: **23.08.2007**

(51) Int Cl.:
*C07C 1/24* (2006.01)      *B01J 29/40* (2006.01)
*C07C 4/02* (2006.01)      *C07C 4/08* (2006.01)
*C07C 11/06* (2006.01)

(86) International application number:
**PCT/JP2007/066395**

(87) International publication number:
**WO 2008/029631 (13.03.2008 Gazette 2008/11)**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **30.08.2006 JP 2006234007
28.09.2006 JP 2006264513**

(71) Applicant: **JGC Corporation
Tokyo 100-0004 (JP)**

(72) Inventors:
• **ITO, Hirofumi
Higashiibaraki-gun, Ibaraki 3111313 (JP)**
• **YOSHIDA, Jiro
Yokohama-shi, Kanagawa 2206001 (JP)**
• **FUNATSU, Shuichi
Yokohama-shi, Kanagawa 2206001 (JP)**
• **OYAMA, Koji
Yokohama-shi, Kanagawa 2206001 (JP)**
• **CHIKAMATSU, Nobuyasu
Yokohama-shi, Kanagawa 2206001 (JP)**

(74) Representative: **Ilgart, Jean-Christophe et al
BREVALEX
3, rue du Docteur Lancereaux
75008 Paris (FR)**

(54) **PROPYLENE PRODUCTION PROCESS AND PROPYLENE PRODUCTION APPARATUS**

(57)      A method for producing propylene including: transferring a feed gas including dimethyl ether and/or methanol and C4 and/or C5 olefins into a reactor, and reacting the feed gas in the presence of a catalyst, the feed gas prior to transferring into the reactor having a (supplied C4 and/or C5 olefins)/(supplied dimethyl ether and methanol) ratio of 0.25 to 7.5, in terms of the molar ratio based on the number of carbon atoms, and the feed gas being contacted with the catalyst at 350°C to 600°C; and an apparatus for producing propylene including: a hydrogenation reactor in which alkynes and/or dienes contained in C4 and/or C5 hydrocarbons is partially hydrogenated to be converted into an olefin having one double bond; a reactor in which C4 and/or C5 hydrocarbons is reacted with dimethyl ether and/or methanol in the presence of a catalyst; and a separator for separating propylene from the reaction product.

FIG. 3

EP 2 058 290 A1

**Description**

TECHNICAL FIELD

The present invention relates to a method and apparatus for producing propylene from dimethyl ether and/or methanol by dehydration-condensation reaction.
Priority is claimed on Japanese Patent Application No. 2006-234007, filed August 30, 2006, and Japanese Patent Application No. 2006-264513, filed September 28, 2006, the contents of which are incorporated herein by reference.

BACKGROUND ART

Presently, due to the difference between ethylene and propylene with respect to the increase in demand, and the increase in the supply by construction of ethane crackers in the Middle East, there is an increasing need for a selective, increased production process of propylene.
Propylene is mostly produced as a by-product produced from an apparatus using a raw material derived from crude oil, such as a naphtha cracker or a fluid catalytic cracking (FCC) apparatus. In the production process using such an apparatus, fractions containing large amounts of olefins of 4 carbon atoms and olefins of 5 carbon atoms are produced. Therefore, an effective technique has been desired which enables conversion of such fractions into propylene.
Conventionally, a method for producing paraffins and aromatic compounds using a FCC apparatus has been disclosed in which ZSM-5 zeolite catalyst is added, and methanol is supplied simultaneously with hydrocarbons (paraffins). In this method, the exothermic dehydration-condensation reaction of methanol is approximately heat balanced by the endothermic catalytic cracking reaction (for example, see Patent Document 1).
Further, a method has been disclosed in which ethylene and aromatic compounds are produced by the catalytic conversion of a mixture of methanol and/or dimethyl ether with hydrocarbons in the presence of a zeolite catalyst.

[Patent Document 1] Japanese Unexamined Patent Application, First Publication No. 62-179592
[Patent Document 2] Japanese Unexamined Patent Application, First Publication No. 60-120790

DISCLOSURE OF INVENTION

PROBLEMS TO BE SOLVED BY THE INVENTION

In the conventional method for producing lower hydrocarbons using a FCC apparatus and the conventional method for producing lower hydrocarbons by thermal cracking of naphtha, propylene was merely a by-product. Therefore, these methods were unsuitable for increased production of only propylene.
Further, in either of the above-mentioned methods, olefins of 4 carbons atoms and olefins of 5 carbon atoms were by-produced in excess amounts. Among olefins of 4 carbon atoms, 2-butene has been used in an increased production of propylene as a raw material for the metathesis reaction. However, the other olefins of 4 carbon atoms and olefins of 5 carbon atoms were cheaply used as raw materials for chemical products.
Furthermore, the above-mentioned by-products contain alkynes and dienes which cause deposition of gum substances and carbon substances. Therefore, when these by-products are directly used as raw materials, it is highly possible that solid deposits are generated and adhered to pipes within the reaction apparatus or on the reaction catalyst, and hence, the pipes are clogged or the reaction catalyst is deactivated.
The present invention takes the above circumstances into consideration, with an object of providing a method for producing propylene in which fractions containing large amounts of olefins of 4 carbon atoms and/or olefins of 5 carbon atoms by-produced in a production process by an apparatus using a raw material derived from crude oil, such as a naphtha cracker or a FCC apparatus. More specifically, an object of the present invention is to provide a method for producing propylene in which olefins of 4 carbon atoms and/or olefins of 5 carbon atoms can be used as raw materials regardless of the isomerism, and achieving conversion into propylene with high selectivity by feeding the raw materials simultaneously with dimethyl ether and/or methanol.
Another object of the present invention is to provide a method and apparatus for producing propylene in which generation of deposits in the pipes of the reaction apparatus and on the reaction catalyst can be suppressed, and clogging of the pipes and deactivation of the reaction catalyst can be prevented.

MEANS TO SOLVE THE PROBLEMS

The present invention provides a method for producing propylene including: transferring a feed gas into a reactor, the feed gas containing at least one member selected from the group consisting of dimethyl ether and methanol and at least one member selected from the group consisting of olefins of 4 carbon atoms and olefins of 5 carbon atoms; and reacting the feed gas in the presence of a catalyst, the feed gas prior to transferring into the reactor having a ratio of the total supplied quantity of olefins of 4 carbon atoms and olefins of 5 carbon atoms to the total supplied quantity of dimethyl ether and methanol within the range from 0.25 to 7.5, in terms of the molar ratio based on the number of carbon atoms, and the feed gas being contacted with the catalyst at a temperature of 350°C to 600°C.

It is preferable that the at least one member selected from the group consisting of olefins of 4 carbon atoms and olefins of 5 carbon atoms includes a product obtained by producing an olefin using an olefin production device and subjecting the olefin to separation using a separator.

More preferably, the present invention provides a method for producing propylene including: transferring a feed gas into a reactor, the feed gas containing at least one member selected from the group consisting of dimethyl ether and methanol and at least one member selected from the group consisting of olefins of 4 carbon atoms and olefins of 5 carbon atoms, the at least one member selected from the group consisting of olefins of 4 carbon atoms and olefins of 5 carbon atoms being a product obtained by subjecting an olefin produced using an olefin production device to separation using a separator; and reacting the feed gas in the presence of a catalyst, the feed gas prior to transferring into the reactor having a ratio of the total supplied quantity of olefins of 4 carbon atoms and olefins of 5 carbon atoms to the total supplied quantity of dimethyl ether and methanol within the range from 0.25 to 7.5, in terms of the molar ratio based on the number of carbon atoms, and the feed gas being contacted with the catalyst at a temperature of 350°C to 600°C, and the reaction product obtained in the reactor and containing propylene as a main component is supplied back to the separator to separate propylene from other components (hereafter, the above-mentioned method is frequently referred to as "the first embodiment of the method for producing propylene according to the present invention").

The above-mentioned olefin production device may be a thermal-cracking device for hydrocarbons and/or a catalytic-cracking device for hydrocarbons. Alternatively, the olefin production device may be a dimerizing apparatus capable of dimerizing ethylene.

The above-mentioned catalyst is preferably a MFI zeolite catalyst.

The catalyst may inlcude a MFI zeolite catalyst containing an alkaline earth metal, which has a Si/Al molar ratio of 10 to 300 and an alkaline earth metal/Al molar ratio of 0.75 to 15.

Further, the present invention provides a method for producing propylene including: transferring a feed gas comprising into a reactor, the feed gas containing at least one member selected from the group consisting of dimethyl ether and methanol and at least one member selected from the group consisting of hydrocarbons of 4 carbon atoms and hydrocarbons of 5 carbon atoms; and reacting the feed gas in the presence of a reaction catalyst, in which the hydrocarbons of 4 carbon atoms and/or hydrocarbons of 5 carbon atoms includes at least one member selected from the group consisting of alkynes and dienes, and the hydrocarbons of 4 carbon atoms and/or hydrocarbons of 5 carbon atoms has hydrogen supplied thereto to convert the at least one member selected from the group consisting of alkynes and dienes to an olefin having one double bond by partial hydrogenation, and the olefin having one double bond is supplied into the reactor with the at least one member selected from the group consisting of dimethyl ether and methanol (hereafter, the above-mentioned method is sometimes referred to as "the second embodiment of the method for producing propylene according to the present invention").

The hydrocarbons of 4 carbon atoms and/or hydrocarbons of 5 carbon atoms may include a product obtained by producing an olefin using an olefin production device and subjecting the olefin to separation using a separator. Further, the hydrocarbons of 4 carbon atoms and/or hydrocarbons of 5 carbon atoms are preferably hydrocarbons mainly containing olefins.

The olefin production device may be at least one member selected from the group consisting of a thermal-cracking device for hydrocarbons, a catalytic-cracking device for hydrocarbons and a device for subjecting an oxygen-containing hydrocarbon to dehydration-condensation reaction.

Further, at least a part of the hydrogen to be supplied to the hydrocarbons of 4 carbon atoms and/or hydrocarbons of 5 carbon atoms may be produced in the reactor.

The catalyst may be a MFI zeolite catalyst.

The catalyst may include a MFI zeolite catalyst containing an alkaline earth metal, which has a Si/Al molar ratio of 10 to 300 and an alkaline earth metal/Al molar ratio of 0.75 to 15.

The present invention also provides an apparatus for producing propylene including: a hydrogenation reactor in which hydrogen is supplied to at least one member selected from the group consisting of alkynes and dienes contained in hydrocarbons of 4 carbon atoms and/or hydrocarbons of 5 carbon atoms, and the at least one member selected from the group consisting of alkynes and dienes is partially hydrogenated to be converted into an olefin having one double bond; a reactor in which the resulting hydrocarbons of 4 carbon atoms and/or hydrocarbons of 5 carbon atoms obtained in the hydrogenation reactor is reacted with at least one member selected from the group consisting of dimethyl ether and methanol in the presence of a catalyst; and a separator for separating propylene from the reaction product obtained in the reactor.

In the hydrogenation reactor, the partial hydrogenation is performed by using a hydrogenation catalyst containing palladium.

Further, at least a part of the hydrogen to be supplied to the hydrogenation reactor may be produced in the reactor.

EFFECT OF THE INVENTION

The first embodiment of the method for producing propylene according to the present invention includes: transferring a feed gas into a reactor, the feed gas containing at least one member selected from the group consisting of dimethyl ether and methanol and at least one member selected from the group consisting of olefins of 4 carbon atoms and olefins of 5 carbon atoms; and reacting the feed gas in the presence of a catalyst, the feed gas prior to transferring into the reactor having a ratio of the total supplied quantity of olefins of 4 carbon atoms and olefins of 5 carbon atoms to the total supplied quantity of dimethyl ether and methanol within the range from 0.25 to 7.5, in terms of the molar ratio based on the number of carbon atoms, and the feed gas being contacted with the catalyst at a temperature of 350°C to 600°C. By transferring olefins of 4 carbon atoms and olefins of 5 carbon atoms into the reactor, the selectivity for the desired product such as propylene can be enhanced, and hence, the final yield of the desired product can be enhanced.

More preferably, the first embodiment of the method for producing propylene according to the present invention includes: transferring a feed gas into a reactor, the feed gas containing at least one member selected from the group consisting of dimethyl ether and methanol and at least one member selected from the group consisting of olefins of 4 carbon atoms and olefins of 5 carbon atoms, the at least one member selected from the group consisting of olefins of 4 carbon atoms and olefins of 5 carbon atoms being a product obtained by subjecting an olefin produced using an olefin production device to separation using a separator; and reacting the feed gas in the presence of a catalyst, the feed gas prior to transferring into the reactor having a ratio of the total supplied quantity of olefins of 4 carbon atoms and olefins of 5 carbon atoms to the total supplied quantity of dimethyl ether and methanol within the range from 0.25 to 7.5, in terms of the molar ratio based on the number of carbon atoms, and the feed gas being contacted with the catalyst at a temperature of 350°C to 600°C, and the reaction product obtained in the reactor and containing propylene as a main component is supplied back to the separator to separate propylene, olefins of 4 carbon atoms and olefins of 5 carbon atoms and other components, and transferring the separated olefins of 4 carbon atoms and olefins of 5 carbon atoms to the reactor. As a result, in the entire process, the selectivity for the desired product such as propylene can be enhanced, and hence, the final yield of the desired product can be enhanced.

In the second embodiment of the method for producing propylene according to the present invention, the alkynes and/or dienes contained in the feed gas to be supplied to the reactor for producing propylene can be converted into olefins having one double bond by partial hydrogenation. By converting alkynes and/or dienes into olefins having one double bond by using a hydrogenation reactor, the content of alkynes and/or dienes in the feed gas to be supplied to the reactor can be reduced to an extremely small amount.

The hydrogenation reaction may be either a liquid-phase reaction or a gaseous-phase reaction.

Further, by converting alkynes and/or dienes into olefins having one double bond by using a hydrogenation reactor to reduce the alkynes and/or dienes contained in the feed gas to be supplied to the reactor to an extremely small amount, solids containing carbon can be prevented from depositing in pipes connected to the reactor and on the catalyst. As a result, it becomes possible to reliably prevent various problems, such as clogging of the pipes connected to the reactor by the deposit, and deactivation of the reaction catalyst caused by adhering of the deposit on the surface thereof.

BRIEF DESCRIPTION OF THE DRAWINGS

FIG. 1 is a schematic diagram showing the flow of one mode of the first embodiment of the method for producing propylene according to the present invention.
FIG. 2 is a graph showing the relation between the reaction temperature (°C) and the yield of methane (weight %).
FIG.3 is an explanatory diagram showing an example of the apparatus for producing propylene according to the present invention and the method using the same (the second embodiment of the method for producing propylene according to the present invention).

REFERENCE NUMERALS

2    Reactor
4    Separator
6    Olefin production device

10    Apparatus for producing propylene
11    Olefin production device
12    Hydrogenation reactor
13    Reactor
14    Separator

BEST MODE FOR CARRYING OUT THE INVENTION

First, explanation is given of the best mode of the first embodiment of the method for producing propylene according to the present invention.
The following embodiment is specifically explained only for easier understanding of the principal of the present invention, and the present invention is not to be limited thereto, unless otherwise described.

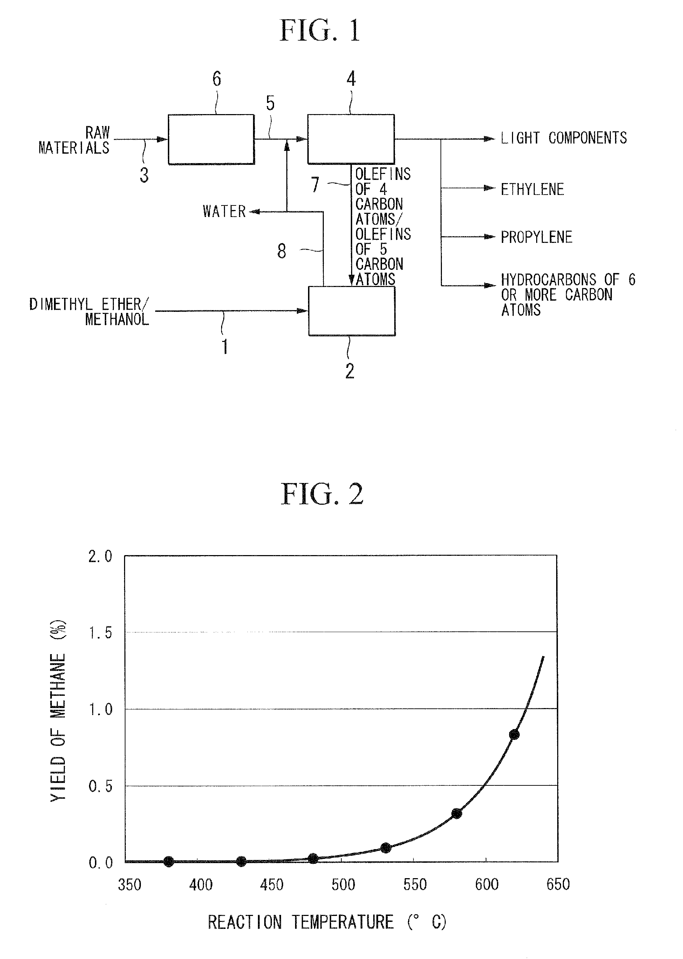

FIG. 1 is a schematic diagram showing the flow of one mode of the first embodiment of the method for producing propylene according to the present invention.
In the present embodiment of the method for producing propylene, a feed gas including at least one member selected from the group consisting of dimethyl ether and methanol and at least one member selected from the group consisting of olefins of 4 carbon atoms and olefins of 5 carbon atoms is transferred to a reactor 2.
Either of dimethyl ether or methanol, or both of dimethyl ether and methanol is/are transferred in a gaseous state from a gas-feeding device (not shown) to the reactor 2 via pipe 1. On the other hand, raw materials are supplied to olefin production device 6 via pipe 3, and a product containing lower olefins is produced by the olefin production device. Then, the product is transferred to separator 4 via pipe 5, and olefins of 4 carbon atoms and/or olefins of 5 carbon atoms are separated therefrom by the separator 4. The separated olefins of 4 carbon atoms and/or olefins of 5 carbon atoms are transferred to reactor 2 via pipe 7.
The at least one member selected from the group consisting of dimethyl ether and methanol may contain any other gases such as steam, nitrogen, argon and carbon dioxide.
Further, in the present embodiment of the method for producing propylene, the feed gas prior to transferring into the reactor has a ratio of the total supplied quantity of olefins of 4 carbon atoms and olefins of 5 carbon atoms to the total supplied quantity of dimethyl ether and methanol within the range from 0.25 to 7.5, preferably from 1.0 to 6.0, in terms of the molar ratio based on the number of carbon atoms.
Herein, the "molar ratio based on the number of carbon atoms" is a value calculated from the following formula:
(C4 Mol Flow Rate $\times$ 4 + C5 Mol Flow Rate $\times$ 5)/(DME Mol Flow Rate $\times$ 2 + MeOH Mol Flow Rate $\times$ 1)
When the ratio of the total supplied quantity of olefins of 4 carbon atoms and olefins of 5 carbon atoms to the total supplied quantity of dimethyl ether and methanol supplied is less than 0.25, in terms of the molar ratio based on the number of carbon atoms, the temperature elevation caused by the exothermic reaction within the reactor becomes large, and hence, the temperature at the outlet becomes high. As a result, the catalyst is rapidly deactivated, and production of by-products increases. On the other hand, when the ratio in terms of the molar ratio based on the number of carbon atoms exceeds 7.5, the temperature decrease caused by endothermic reaction within the reactor becomes large, and hence, it becomes necessary to provide a heating device within the reactor or to elevate the temperature of the feed gas. As a result, problems are caused in that the structure of the apparatus becomes complicated, and carbon substances deposit in the feed gas lines.
The inside of the reactor 2 is filled with the catalyst. A reaction such as a dehydration-condensation reaction is effected by the action of the catalyst, and hydrocarbons of no more than 6 carbon atoms such as ethylene, propylene, butene, pentene and hexene are produced as main products.
As the catalyst, a MFI zeolite catalyst, an alkaline earth metal-containing MFI zeolite catalyst, a silico-alumino-phosphate catalyst, or the like is used in a fluidized-bed reaction system, fixed-bed reaction system, moving-bed reaction system, or the like. Among these, MFI zeolite catalysts and alkaline earth metal-containing MFI zeolite catalysts are preferable as they enable lower hydrocarbons to be obtained in high yields.
With respect to the reaction conditions within the reactor 2, the above-mentioned feed gas is contacted with the catalyst at a temperature of 350°C to 600°C. Further, it is preferable that the weight hourly space velocity (hereafter, frequently abbreviated as "WHSV"), which is the weight in terms of the supplied quantity of dimethyl ether (hereafter, frequently abbreviated as "DME"), per unit weight of the catalyst and unit time, be within the range of 0.025 g-DME/(g-catalyst · hour) to 50 g-DME/(g-catalyst · hour). Furthermore, the pressure is preferably within the range of atmospheric pressure to 1 MPa.
When the feed gas is contacted with the catalyst at a temperature lower than 350°C, the production rate of the desired product becomes low, and hence, is not economical. On the other hand, when the feed gas is contacted with the catalyst at a temperature higher than 600°C, the catalyst is rapidly deactivated, and by-products such as methane and the like are produced in large amounts.
When the WHSV is less than 0.025 g-DME/(g-catalyst · hour), the productivity per unit volume of the fixed-bed reactor becomes low, and hence, is not economical.

On the other hand, when the WHSV is more than 50 g-DME/(g-catalyst · hour), the catalyst life and the catalyst activity become unsatisfactory.

By adjusting the reaction conditions within the reactor 2, the content of the desired lower hydrocarbon within the product can be changed. For example, for increasing the content of propylene, it is preferable to lower the reaction pressure.

The product with propylene as a main component obtained in the reactor 2 is transferred to a heat exchanger (not shown) via pipe 8 and cooled. Then, the product is transferred to the separator 4 and separated into, for example, light components such as methane and ethane, ethylene, propylene, olefins of 4 carbon atoms, olefins of 5 carbon atoms, and heavy hydrocarbons of 6 or more carbon atoms.

Among the components separated by the separator 4, olefins of 4 carbon atoms or olefins of 5 carbon atoms are introduced into the reactor 2 via pipe 7. The other components are recovered separately.

In the present embodiment of the method for producing propylene, olefins of 4 carbon atoms or olefins of 5 carbon atoms are separated by the separator 4 and transferred to the reactor 2. As a result, the selectivity for the desired product such as propylene can be enhanced, and hence, the final yield of the desired product can be enhanced.

Further, by supplying a feed gas containing at least one member selected from the group consisting of olefins of 4 carbon atoms and olefins of 5 carbon atoms to the reactor 2, the life of the catalyst for producing propylene from dimethyl ether and/or methanol transferred to the reactor 2 can be increased. The reaction of the olefins of 4 carbon atoms or olefins of 5 carbon atoms within the reactor 2 is generally an endothermic reaction, and suppresses the temperature elevation caused by the exothermic reaction of dimethyl ether and/or methanol within the reactor 2. As a result, the deactivation of the catalyst is suppressed.

Therefore, it becomes possible to reduce the amount of the catalyst to be filled and to increase the cycle of the catalyst recycling. Hence, the costs of equipments and operation can be reduced.

Next, explanation is given of the best mode of the second embodiment of the method for producing propylene and apparatus for producing the same according to the present invention. The following embodiment is specifically explained only for easier understanding of the principal of the present invention, and the present invention is not to be limited thereto, unless otherwise described.

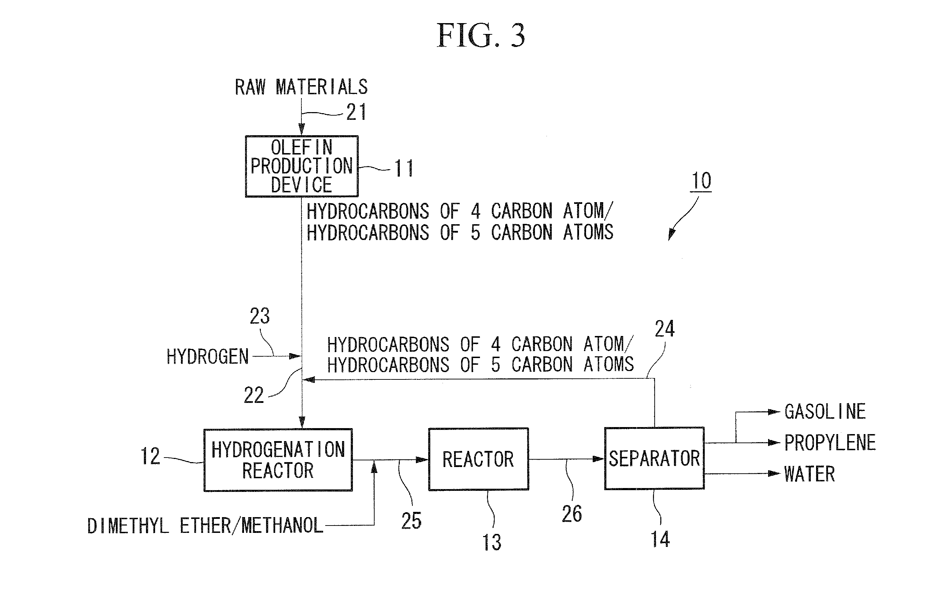

FIG.3 is an explanatory diagram of the apparatus for producing propylene according to the present invention and the method using the same (the second embodiment of the method for producing propylene according to the present invention).

The apparatus 10 for producing propylene according to the present embodiment is provided with an olefin production device 11, a hydrogenation reactor 12, a reactor 13 and a separator 14. In the olefin production device 11, a product containing lower olefins is produced from the supplied raw materials. In the hydrogenation reactor 12, hydrogen is added to hydrocarbons of 4 carbon atoms and/or hydrocarbons of 5 carbon atoms produced in the olefin production device 11 and hydrocarbons of 4 carbon atoms and/or hydrocarbons of 5 carbon atoms refluxed from the separator 14 (described below), and a reaction is effected. In this manner, the alkynes and/or dienes contained in the hydrocarbons of 4 carbon atoms and/or hydrocarbons of 5 carbon atoms are partially hydrogenated so as to be converted into olefins having one double bond.

In the reactor 13, the resulting hydrocarbons of 4 carbon atoms and/or hydrocarbons of 5 carbon atoms obtained in the hydrogenation reactor 12 is reacted with dimethyl ether and/or methanol supplied, so as to produce hydrocarbons including propylene. The separator 14 separates and purifies the hydrocarbons including propylene obtained in the reactor 13, so as to extract the respective components such as propylene, gasoline, water, hydrogen, and hydrocarbons of 4 carbon atoms and/or hydrocarbons of 5 carbon atoms. Of these components, hydrocarbons of 4 carbon atoms and/or hydrocarbons of 5 carbon atoms and hydrogen are refluxed to the hydrogenation reactor 12.

Explanation is given of the second embodiment of the method for producing propylene according to the present invention which uses the apparatus for producing propylene employing such a configuration as described above. Firstly, raw materials are transferred to the olefin production device 11 via pipe 21, and a product containing lower olefins is produced. Then, hydrocarbons of 4 carbon atoms and/or hydrocarbons of 5 carbon atoms are separated from the obtained product by a separation device (not shown). This separation of hydrocarbons of 4 carbon atoms and/or hydrocarbons of 5 carbon atoms may be performed by the separator 14. In such a case, hydrocarbons of 4 carbon atoms and/or hydrocarbons of 5 carbon atoms are transferred to the hydrogenation reactor 12 via pipe 24. In this manner, only one separator is used in the entire apparatus for producing propylene, and hence, the apparatus and process can be simplified.

Subsequently, the hydrocarbons of 4 carbon atoms and/or hydrocarbons of 5 carbon atoms separated from the product obtained in the olefin production device 11 is transferred to the hydrogenation reactor 12 via pipe 22. On the other hand, hydrogen is transferred to the hydrogenation reactor 12 via pipe 23. At least a part of the hydrogen may be produced in the reactor 13 and refluxed to the hydrogenation reactor 12 via pipe 24. Further, the hydrocarbons of 4 carbon atoms and/or hydrocarbons of 5 carbon atoms separated by the separated 14 may be transferred to the hydrogenation reactor 12 via pipe 24.

The hydrocarbons of 4 carbon atoms and/or hydrocarbons of 5 carbon atoms and hydrogen transferred to the hydrogenation reactor 12 are reacted, so as to convert the alkynes and/or dienes contained in the hydrocarbons of 4 carbon atoms and/or hydrocarbons of 5 carbon atoms into olefins having one double bond by partial hydrogenation. The inside of the hydrogenation reactor 12 is filled with a hydrogenation catalyst, and olefins having one double bond are produced as main products by the activity of the hydrogenation catalyst. As the hydrogenation catalyst, a catalyst containing palladium can be preferably used.

Thereafter, the resulting hydrocarbons of 4 carbon atoms and/or hydrocarbons of 5 carbon atoms obtained in the hydrogenation reactor 12 (in which the alkynes and/or dienes are converted into olefins having one double bond by partial hydrogenation) and at least one member selected from the group consisting of dimethyl ether and methanol are transferred to the reactor 13 via pipe 25. The at least one member selected from the group consisting of dimethyl ether and methanol may contain any other gases such as steam, methane, ethane, nitrogen, argon and carbon dioxide.

The inside of the reactor 13 is filled with a reaction catalyst. A reaction such as a dehydration-condensation reaction or catalytic cracking is effected by the activity of the catalyst, and propylene is obtained. Further, lower hydrocarbons of no more than 6 carbon atoms such as ethylene, butene, pentene and hexene, water, and a small amout of hydrogen are also obtained as main products.

As the reaction catalyst used in the reactor 13, a MFI zeolite catalyst, an alkaline earth metal-containing MFI zeolite catalyst, a silico-alumino-phosphate catalyst, or the like is used in a fluidized-bed reaction system, fixed-bed reaction system, moving-bed reaction system, or the like. Among these, a MFI zeolite catalyst s is preferable as they enable lower hydrocarbons to be obtained in high yields, and an alkaline earth metal-containing MFI zeolite catalyst is more preferable.

As an example of the reaction conditions within the reactor 13, the above-mentioned feed gas may be contacted with the catalyst at a temperature of 350°C to 600°C. Further, it is preferable that the weight hourly space velocity (hereafter, frequently abbreviated as "WHSV"), which is the weight in terms of the supplied quantity of dimethyl ether (hereafter, frequently abbreviated as "DME"), per unit weight of the catalyst and unit time be within the range of 0.025 g-DME/(g-catalyst · hour) to 50 g-DME/(g-catalyst · hour). Furthermore, the pressure is preferably in the range of atmospheric pressure to 1 MPa.

When the feed gas is contacted with the catalyst at a temperature lower than 350°C, the production rate of the desired product becomes low, and hence, is not economical. On the other hand, when the feed gas is contacted with the catalyst at a temperature higher than 600°C, the catalyst is rapidly deactivated, and by-products such as methane and the like are produced in large amounts.

When the WHSV is less than 0.025 g-DME/(g-catalyst · hour), the productivity per unit volume of the fixed-bed reactor becomes low, and hence, is not economical.

On the other hand, when the WHSV is more than 50 g-DME/(g-catalyst · hour), the catalyst life and the catalyst activity become unsatisfactory.

By adjusting the reaction conditions within the reactor 13, the content of the desired lower hydrocarbon within the product can be changed. For example, for increasing the content of propylene, it is preferable to lower the reaction pressure.

The product with propylene as a main component obtained in the reactor 13 is transferred to a heat exchanger (not shown) via pipe 26 and cooled. Then, the product is transferred to the separator 14 and separated into, for example, light components such as methane and ethane, ethylene, propylene, olefins of 4 carbon atoms, olefins of 5 carbon atoms, and heavy hydrocarbons of 6 or more carbon atoms.

Among the components separated by the separator 14, olefins of 4 carbon atoms or olefins of 5 carbon atoms are refluxed to the hydrogenation reactor 12 via pipe 24. The other components are recovered separately.

According to the apparatus and method for producing propylene employing the configuration as described above, the alkynes and/or dienes contained in the hydrocarbons of 4 carbon atoms and/or hydrocarbons of 5 carbon atoms to be supplied to the reactor 13 can be converted into olefins having one double bond by partial hydrogenation. By converting the alkynes and/or dienes into olefins having one double bond by the hydrogenation reactor 12, the content of the alkynes and/or dienes in the feed gas to be supplied to the reactor 13 can be reduced to an extremely small amount.

When the feed gas contains alkynes and/or dienes in large amounts, there is a possibility that solid deposits containing carbon are generated and adhered to the inside of the pipes connected to the reactor and on the reaction catalyst. Especially when the ratio of the total supplied quantity of the hydrocarbons of 4 carbon atoms and/or hydrocarbons of 5 carbon atoms to the total supplied quantity of dimethyl ether and methanol becomes large, the temperature decrease caused by endothermic reaction within the reactor becomes large, and hence, it becomes necessary to elevate the temperature of the feed gas. However, when the temperature of the feed gas is elevated, it becomes highly possible that such solid deposits containing carbon are generated.

On the other hand, in the present invention, alkynes and/or dienes are converted into olefins having one double bond by the hydrogenation reactor 12, and the content of the alkynes and/or dienes in the feed gas to be supplied to the reactor 13 is reduced to an extremely small amount. As a result, solids containing carbon can be prevented from depositing in the pipes connected to the reactor 13 and on the reaction catalyst. Hence, it becomes possible to reliably prevent various problems, such as clogging of the pipes connected to the reactor 13 by the deposit, and deactivation of the reaction catalyst filled in the reactor 13 caused by adhering of the deposit thereon.

Further, in the present embodiment, hydrocarbons of 4 carbon atoms and/or hydrocarbons of 5 carbon atoms are separated by the separator 14, and refluxed to the reactor 13 via the hydrogenation reactor 12. As a result, in the entire process, the selectivity for the desired product such as propylene can be enhanced, and hence, the final yield of the desired product can be enhanced.

In addition, by supplying a feed gas containing at least one member selected from the group consisting of hydrocarbons of 4 carbon atoms and/or hydrocarbons of 5 carbon atoms to the reactor 13, the life of the reaction catalyst for producing propylene from dimethyl ether and/or methanol transferred to the reactor 13 can be increased. The hydrocarbons of 4 carbon atoms and/or hydrocarbons of 5 carbon atoms are preferably hydrocarbons mainly containing olefins.

The reaction of the hydrocarbons of 4 carbon atoms and/or hydrocarbons of 5 carbon atoms mainly containing olefins within the reactor 13 is generally an endothermic reaction, and suppresses the temperature elevation caused by the exothermic reaction of dimethyl ether and/or methanol within the reactor 13. As a result, the deactivation of the catalyst is suppressed. Therefore, it becomes possible to reduce the amount of the catalyst to be filled, and increase the cycle of the catalyst recycling. Hence, the costs of equipments and operation can be reduced.

EXAMPLES

Hereinafter, the present invention will be described in more detail by reference to examples, which are, however, not to be construed as limiting the invention.

Example A

[Preparation of catalyst]

<Catalyst Preparation Example 1>

A calcium-containing MFI zeolite was prepared in accordance with the preparation method described in Japanese Unexamined Patent Application, First Publication No. 2005-138000.

Subsequently, using hydrochloric acid, the prepared catalyst was converted into a proton type by a typical operation. Then, the resulting catalyst was dried at 120°C for 5 hours, followed by calcination in air at 520°C for 10 hours, thereby obtaining a proton-type, calcium-containing MFI-structured zeolite catalyst.

In the actual use, the catalyst was prepared either by subjecting to compression molding without using a binder, followed by refining the particle size (hereafter, this catalyst is referred to as "HCaMFI-A catalyst"), or by molding with alumina as a binder (hereafter, this catalyst is referred to as "HCaMFI-B catalyst").

<Catalyst Preparation Example 2>

An ammonium-type MFI zeolite (manufactured by Zeolyst Corp.) having a Si/Al molar ratio of 80 was calcined at 530°C for 6 hours, thereby obtaining a HMFI catalyst.

In the actual use, the catalyst was prepared by subjecting to compression molding without using a binder, followed by refining the particle size.

[Synthesis of lower hydrocarbons]

Using the HCaMFI-A catalyst, the HCaMFI-B catalyst or the HMFI catalyst, lower hydrocarbons were synthesized from dimethyl ether only, or dimethyl ether and isobutene.

When hydrocarbons were synthesized from dimethyl ether and isobutene as raw materials, the yield (weight %) of each hydrocarbon was determined by formula (1) shown below. Further, the conversion (weight %) of isobutene was determined by formula (2) shown below.

$$Yi = (Ri - RDMEi)/FC4 \times 100 \qquad (1)$$

wherein Yi represents the yield of the lower hydrocarbon from isobutene (component (i)); Ri represents the mass flow rate of the component (i) at the outlet of the reactor when dimethyl ether and isobutene were used as raw materials; RDMEi represents the mass flow rate of the component (i) at the outlet of the reactor when only dimethyl ether was used as the raw material; and FC4 represents the mass flow rate of isobutene at the inlet of the reactor.

$$\text{Conv.} = 100 - \text{YC4}$$

wherein Conv. represents the conversion of isobutene; and YC4 represents the yield of hydrocarbons having 4 carbon atoms produced from isobutene.

<Test Example 1A>

Using a HCaMFI-A catalyst, lower hydrocarbons were synthesized from dimethyl ether by an isothermal reactor, as follows.
Dimethyl ether and nitrogen were mixed together at flow rates of 1,291 Ncm$^3$/hour and 1,291 Ncm$^3$/hour, respectively. Then, the resulting mixture was transferred to an isothermal reactor, and reacted with a catalyst at 530°C under atmospheric pressure. The weight hourly space velocity (WHSV), which is the ratio of the supplied quantity of dimethyl ether (DME) as a raw material to the quantity of the catalyst, was set to be 9.6 g-DME/(g-catalyst · hour).
The yields (weight %) of methane, hydrocarbons of 2 carbon atoms, propylene, propane, hydrocarbons of 4 carbon atoms and hydrocarbons of 5 carbon atoms are shown in Table 1.
In Table 1, "C2" indicates that the number of carbon atoms is 2, "C4" indicates that the number of carbon atoms is 4, and "C5+" indicates that the number of carbon atoms is 5 or more.

<Test Example 2A>

Lower hydrocarbons were synthesized from dimethyl ether in substantially the same manner as in Comparative Example 1A, except that a HCaMFI-B catalyst was used, dimethyl ether and nitrogen were mixed together at flow rates of 448 Ncm$^3$/hour and 448 Ncm$^3$/hour, respectively, and the weight hourly space velocity (WHSV) was set to be 3.3 g-DME/(g-catalyst · hour).
The yields (weight %) of methane, hydrocarbons of 2 carbon atoms, propylene, propane, hydrocarbons of 4 carbon atoms and hydrocarbons of 5 carbon atoms are shown in Table 1.

<Example 1A>

Using a HCaMFI-A catalyst, lower hydrocarbons were synthesized from dimethyl ether and isobutene by an isothermal reactor, as follows.
Dimethyl ether, isobutene and nitrogen were mixed together at flow rates of 1,279 Ncm$^3$/hour, 732 Ncm$^3$/hour and 1,279 Ncm$^3$/hour, respectively. Then, the resulting mixture was transferred to an isothermal reactor, and reacted with a catalyst at 530°C under atmospheric pressure. The weight hourly space velocity (WHSV), which is the ratio of the supplied quantity of dimethyl ether (DME) as a raw material to the quantity of the catalyst, was set to be 9.5 g-DME/(g-catalyst · hour).
The yields (weight %) of methane, hydrocarbons of 2 carbon atoms, propylene, propane, hydrocarbons of 4 carbon atoms and hydrocarbons of 5 carbon atoms, and the conversion (weight %) of isobutene are shown in Table 1.

<Example 2A>

Using a HCaMFI-B catalyst, lower hydrocarbons were synthesized from dimethyl ether and isobutene by an isothermal reactor, as follows.
Dimethyl ether, isobutene and nitrogen were mixed together at flow rates of 448 Ncm$^3$/hour, 441 Ncm$^3$/hour and 448 Ncm$^3$/hour, respectively. Then, the resulting mixture was transferred to an isothermal reactor, and reacted with a catalyst at 530°C under atmospheric pressure. The weight hourly space velocity (WHSV), which is the ratio of the supplied quantity of dimethyl ether (DME) as a raw material to the quantity of the catalyst, was set to be 4.0 g-DME/(g-catalyst · hour).
The yields (weight %) of methane, hydrocarbons of 2 carbon atoms, propylene, propane, hydrocarbons of 4 carbon atoms and hydrocarbons of 5 carbon atoms, and the conversion (weight %) of isobutene are shown in Table 1.

<Example 3A>

Lower hydrocarbons were synthesized from dimethyl ether in substantially the same manner as in Example 2A, except that dimethyl ether, isobutene and nitrogen were mixed together at flow rates of 448 Ncm$^3$/hour, 1,348 Ncm$^3$/hour, and 448 Ncm$^3$/hour, respectively.
The yields (weight %) of methane, hydrocarbons of 2 carbon atoms, propylene, propane, hydrocarbons of 4 carbon atoms and hydrocarbons of 5 carbon atoms, and the conversion (weight %) of isobutene are shown in Table 1.

<Example 4A>

Lower hydrocarbons were synthesized from dimethyl ether in substantially the same manner as in Example 2A, except that a HMFI catalyst was used, dimethyl ether, isobutene and nitrogen were mixed together at flow rates of 448 Ncm$^3$/hour, 441 Ncm$^3$/hour, and 448 Ncm$^3$/hour, respectively , and the reaction temperature was changed to 470°C.
The yields (weight %) of methane, hydrocarbons of 2 carbon atoms, propylene, propane, hydrocarbons of 4 carbon atoms and hydrocarbons of 5 carbon atoms, and the conversion (weight %) of isobutene are shown in Table 1.

<Example 5A>

Lower hydrocarbons were synthesized from dimethyl ether in substantially the same manner as in Example 2A, except that dimethyl ether, isobutene, nitrogen and water were mixed together at flow rates of 1,457 Ncm$^3$/hour, 672 Ncm$^3$/hour, 448 Ncm$^3$/hour and 1,480 Ncm$^3$/hour, respectively.
The yields (weight %) of methane, hydrocarbons of 2 carbon atoms, propylene, propane, hydrocarbons of 4 carbon atoms and hydrocarbons of 5 carbon atoms, and the conversion (weight %) of isobutene are shown in Table 1.

<Comparative Example 1A>

Lower hydrocarbons were synthesized from dimethyl ether in substantially the same manner as in Example 2A, except that dimethyl ether was not supplied, and isobutene, nitrogen and water were mixed together at flow rates of 672 Ncm$^3$/hour, 448 Ncm$^3$/hour and 1,480 Ncm$^3$/hour, respectively.
The yields (weight %) of methane, hydrocarbons of 2 carbon atoms, propylene, propane, hydrocarbons of 4 carbon atoms and hydrocarbons of 5 carbon atoms, and the conversion (weight %) of isobutene are shown in Table 1.
[Table 1]

| | | Test Example 1A | Test Example 2A | Example 1A | Example 2A | Example 3A | Example 4A | Example 5A | Comparative Example 1A |
|---|---|---|---|---|---|---|---|---|---|
| Catalyst | | HCaMF1-A | HCaMF1-B | HCaMFl-A | HCaMF1-B | HCaMF1-A | HMF 1 | HCaMF1-B | HCaMF1-B |
| Temperature | [°C] | 530 | 530 | 530 | 530 | 530 | 470 | 530 | 530 |
| Flow rate of feed gas [mmol/h] | Isobutene | 0. 0 | 0.0 | 32. 7 | 19. 7 | 60. 2 | 20. 1 | 60. 6 | 60. 6 |
| | Dimethyl ether | 57. 6 | 20.0 | 57. 1 | 20. 0 | 20. 0 | 20. 0 | 60. 0 | 0. 0 |
| | Water | 0. 0 | 0.0 | 0. 0 | 0. 0 | 0. 0 | 0. 0 | 66. 5 | 66. 5 |
| | Nitrogen | 57. 6 | 20.0 | 57. 5 | 20. 0 | 20. 0 | 27. 1 | 30. 6 | 30. 6 |
| | Total amount | 115. 2 | 40.0 | 147. 3 | 59. 7 | 100. 2 | 67. 2 | 217. 7 | 157. 7 |
| C4 hydrocarbon/ dimethyl esther | mol-C/mol-C | 0. 0 | 0.0 | 1. 1 | 2. 0 | 6. 0 | 2. 0 | 2. 0 | |
| Product distrubution at outlet of reactor (weight %) | Methane | 0. 9 | 0.5 | 0. 3 | 0. 2 | 0. 1 | 0. 6 | 0. 2 | 0. 0 |
| | C2 hydrocarbon | 5. 2 | 8.4 | 5. 0 | 7. 2 | 5. 5 | 7. 3 | 3. 7 | 2. 2 |
| | Propylene | 42. 3 | 40.0 | 36. 2 | 32. 7 | 28. 5 | 25. 3 | 30. 2 | 21. 7 |
| | Propane | 0. 6 | 1.4 | 0. 8 | 1. 4 | 1. 2 | 5. 0 | 0. 6 | 0. 4 |
| | C4 hydrocarbon | 25. 8 | 26.3 | 31. 3 | 31. 1 | 34. 0 | 31. 3 | 31. 3 | 52. 0 |
| | C5+ hydrocarbon | 25. 2 | 23.3 | 26. 4 | 27. 5 | 30. 6 | 30. 5 | 34. 0 | 23. 6 |

(continued)

| | | Test Example 1A | Test Example 2A | Example 1A | Example 2A | Example 3A | Example 4A | Example 5A | Comparative Example 1A |
|---|---|---|---|---|---|---|---|---|---|
| Catalyst | | HCaMF1-A | HCaMF1-B | HCaMFl-A | HCaMF1-B | HCaMF1-A | HMF 1 | HCaMF1-B | HCaMF1-B |
| Distribution of reaction products from isobutene (weight %) | Mthane | | | 0. 0 | 0. 1 | 0. 1 | 0. 0 | 0. 0 | 0. 0 |
| | C2 hydrocarbon | | | 4. 9 | 6. 6 | 5. 0 | 6. 3 | 3. 9 | 2. 2 |
| | Propylene | | | 30. 8 | 28. 9 | 26. 6 | 28. 7 | 25. 1 | 21. 7 |
| | Propane | | | 0. 9 | 1. 4 | 1. 1 | 1. 2 | 0. 6 | 0. 4 |
| | C4 hydrocarbon | | | 36. 2 | 33. 5 | 35. 3 | 34. 5 | 35. 4 | 52. 0 |
| | C5+ hydrocarbon | | | 27. 5 | 29. 6 | 31. 8 | 29. 3 | 35. 0 | 23. 6 |
| Conversion of isobutene (weight %) | | | | 63. 8 | 66. 5 | 64. 7 | 65. 4 | 64. 5 | 45. 7 |

The distribution of the reaction product from isobutene in each of Examples 1A to 5A as shown in Table 1 was determined by subtracting the experimental result (product distribution) of dimethyl ether alone as shown in Test Example 1A or 2A from the product distribution at the outlet of the reactor. The distribution of the reaction product indicates the yields (weight %) of the desired components from isobutene.

From the results shown in Table 1, it was found that in Examples 1A to 5A, the conversion of isobutene became about 63 to 67 weight % by simultaneously transferring dimethyl ether and isobutene to the reactor.

In Comparative Example 1A, only isobutene was transferred to the reactor. As a result, it was found that the conversion of isobutene was as low as about 46 weight %.

Therefore, from the results of Examples 1A to 5A and Comparative Example 1A, it was found that simultaneous feeding of dimethyl ether and an olefin is effective in enhancing the conversion of the olefin.

Further, when only isobutene was transferred through a pipe preheated to 500°C, it was found that isobutene polymerized within the pipe, such that the pipe was clogged, and synthesis reaction of lower hydrocarbons using isobutene could not be performed.

<Example 6A>

Using a HCaMFI-B catalyst, lower hydrocarbons were synthesized from dimethyl ether and isobutene by an adiabatic reactor, as follows.

Dimethyl ether and hydrocarbons of 4 carbon atoms and/or hydrocarbons of 5 carbon atoms were mixed together at flow rates of 13 g/hour and 37.9 g/hour, respectively. Then, the resulting mixture was transferred to an adiabatic reactor and reacted with a catalyst under conditions wherein the temperature at the inlet of the reactor was 530°C and the pressure was atmospheric pressure.

With respect to the catalyst bed provided within the adiabatic reactor, the temperature (°C) at the inlet and the temperature (°C) at the outlet were measured, and the temperature difference was determined. The results are shown in Table 2.

Further, the yields (weight %) of hydrocarbons of 1 carbon atom, hydrocarbons of 2 carbon atoms, propylene, propane and hydrocarbons of 6 or more carbon atoms are shown in Table 2.

In Table 2, "C1" indicates that the number of carbon atoms is 1, "C2" indicates that the number of carbon atoms is 2, "C4" indicates that the number of carbon atoms is 4, "C5" indicates that the number of carbon atoms is 5, and "C6+" indicates that the number of carbon atoms is 6 or more.

Furthermore, the ratios of the supplied quantities of olefins of 4 carbon atoms, paraffins of 4 carbon atoms, olefins of 5 carbon atoms and paraffins of 5 carbon atoms are also shown in Table 2.

<Example 7A>

Lower hydrocarbons were synthesized from dimethyl ether and isobutene in substantially the same manner as in Example 6A, except that dimethyl ether and hydrocarbons of 4 carbon atoms and/or hydrocarbons of 5 carbon atoms were mixed together at flow rates of 18 g/hour and 38.7 g/hour, respectively, and the temperature at the inlet of the reactor was changed to 501°C.

With respect to the catalyst bed provided within the adiabatic reactor, the temperature (°C) at the inlet and the temperature (°C) at the outlet were measured, and the temperature difference was determined. The results are shown in Table 2.

Further, the yields (weight %) of hydrocarbons of 1 carbon atom, hydrocarbons of 2 carbon atoms, propylene, propane and hydrocarbons of 6 or more carbon atoms are also shown in Table 2.

<Example 8A>

Lower hydrocarbons were synthesized from dimethyl ether and isobutene in substantially the same manner as in Example 6A, except that dimethyl ether and hydrocarbons of 4 carbon atoms and/or hydrocarbons of 5 carbon atoms were mixed together at flow rates of 34 g/hour and 41.1 g/hour, respectively, and the temperature at the inlet of the reactor was changed to 435°C.

With respect to the catalyst bed provided within the adiabatic reactor, the temperature (°C) at the inlet and the temperature (°C) at the outlet were measured, and the temperature difference was determined. The results are shown in Table 2.

Further, the yields (weight %) of hydrocarbons of 1 carbon atom, hydrocarbons of 2 carbon atoms, propylene, propane and hydrocarbons of 6 or more carbon atoms are also shown in Table 2.

<Example 9A>

Lower hydrocarbons were synthesized from dimethyl ether and isobutene in substantially the same manner as in Example 6A, except that dimethyl ether and hydrocarbons of 4 carbon atoms and/or hydrocarbons of 5 carbon atoms were mixed together at flow rates of 55 g/hour and 44.7 g/hour, respectively, and the temperature at the inlet of the reactor was changed to 382°C.
With respect to the catalyst bed provided within the adiabatic reactor, the temperature (°C) at the inlet and the temperature (°C) at the outlet were measured, and the temperature difference was determined. The results are shown in Table 2. Further, the yields (weight %) of hydrocarbons of 1 carbon atom, hydrocarbons of 2 carbon atoms, propylene, propane and hydrocarbons of 6 or more carbon atoms are also shown in Table 2.

<Example 10A>

Lower hydrocarbons were synthesized from dimethyl ether and isobutene in substantially the same manner as in Example 6A, except that dimethyl ether and hydrocarbons of 4 carbon atoms and/or hydrocarbons of 5 carbon atoms were mixed together at flow rates of 98 g/hour and 51.2 g/hour, and the temperature at the inlet of the reactor was changed to 379°C.
With respect to the catalyst bed provided within the adiabatic reactor, the temperature (°C) at the inlet and the temperature (°C) at the outlet were measured, and the temperature difference was determined. The results are shown in Table 2. Further, the yields (weight %) of hydrocarbons of 1 carbon atom, hydrocarbons of 2 carbon atoms, propylene, propane and hydrocarbons of 6 or more carbon atoms are also shown in Table 2.
[Table 2]

| | | Example 6A | Example 7A | Example 8A | Example 9A | Example 10A |
|---|---|---|---|---|---|---|
| Feed fbw rate of dimethylether (g/hour) | | 13 | 18 | 34 | 55 | 98 |
| Feed flow rate of C4/C5 to reactor (g/hour) | C4 olefin | 27.0 | 27.7 | 29.6 | 32.3 | 37.4 |
| | C4 paraffin | 1.4 | 1.4 | 1.5 | 1.8 | 2.2 |
| | C5 olefin | 9.0 | 9.1 | 9.5 | 10.0 | 10.9 |
| | C 5 paraffin | 0.5 | 0.5 | 0.5 | 0.6 | 0.7 |
| | Totalam ount | 37.9 | 38.7 | 41.1 | 44.7 | 51.2 |
| (C4+C5)/DME ratio (mol-c/mol-c) | | 4.73 | 3.53 | 1.98 | 1.33 | 0.86 |
| Temperature at the inlet of catalyst bed [°C] | | 530 | 501 | 435 | 382 | 379 |
| Temperature at the outlet of catalyst bed [°C] | | 531 | 530 | 530 | 531 | 580 |
| Temperature difference betw een inlet and outlet of catalyst bed [°C] | | 1 | 29 | 95 | 149 | 201 |
| Hydrocarbon production ratio (weight%) | C1/C2 hydrocarbon | 17.8 | 17.9 | 18.3 | 18.6 | 19.0 |
| | Propylene | 56.3 | 56.9 | 58.3 | 59.4 | 60.6 |
| | Propane | 1.2 | 1.3 | 1.4 | 1.5 | 1.6 |
| | C 6+ hydrocarbon | 24.7 | 23.9 | 22.0 | 20.5 | 18.8 |

From the results shown in Table 2, in Examples 6A to 10A, it was found that the difference between the temperature (°C) of the catalyst bed at the inlet and the temperature (°C) of the catalyst bed at the outlet becomes larger as the ratio of the supplied quantity of the hydrocarbons of 4 carbon atoms and/or hydrocarbons of 5 carbon atoms to the supplied quantity of dimethyl ether is decreased. In other words, it was confirmed that the total calorific value of the heat generated by the reaction becomes larger as the above-mentioned ratio becomes smaller.

<Test Example 3A>

Using a HCaMFI-A catalyst, lower hydrocarbons were synthesized from dimethyl ether and isobutene by an isothermal reactor, as follows.

Dimethyl ether, isobutene and nitrogen were mixed together at flow rates of 1,279 Ncm$^3$/hour, 732 Ncm$^3$/hour and 1,279 Ncm$^3$/hour, respectively. Then, the resulting mixture was transferred to an isothermal reactor, and reacted with a catalyst at 380°C under atmospheric pressure. The weight hourly space velocity (WHSV), which is the ratio of the supplied quantity of dimethyl ether (DME) as a raw material to the quantity of the catalyst, was set to be 4.1 g-DME/(g-catalyst · hour). The yields (weight %) of methane, hydrocarbons of 2 carbon atoms, propylene, propane, hydrocarbons of 4 carbon atoms and hydrocarbons of 5 or more carbon atoms are shown in Table 3.

<Test Example 4A>

Lower hydrocarbons were synthesized from dimethyl ether and isobutene in substantially the same manner as in Test Example 3A, except that the reaction temperature was changed to 430°C.
The yields (weight %) of methane, hydrocarbons of 2 carbon atoms, propylene, propane, hydrocarbons of 4 carbon atoms and hydrocarbons of 5 or more carbon atoms are shown in Table 3.

<Test Example 5A>

Lower hydrocarbons were synthesized from dimethyl ether and isobutene in substantially the same manner as in Test Example 3A, except that the reaction temperature was changed to 480°C.
The yields (weight %) of methane, hydrocarbons of 2 carbon atoms, propylene, propane, hydrocarbons of 4 carbon atoms and hydrocarbons of 5 or more carbon atoms are shown in Table 3.

<Test Example 6A>

Lower hydrocarbons were synthesized from dimethyl ether and isobutene in substantially the same manner as in Test Example 3A, except that the reaction temperature was changed to 530°C.
The yields (weight %) of methane, hydrocarbons of 2 carbon atoms, propylene, propane, hydrocarbons of 4 carbon atoms and hydrocarbons of 5 or more carbon atoms are shown in Table 3.

<Test Example 7A>

Lower hydrocarbons were synthesized from dimethyl ether and isobutene in substantially the same manner as in Test Example 3A, except that the reaction temperature was changed to 580°C.
The yields (weight %) of methane, hydrocarbons of 2 carbon atoms, propylene, propane, hydrocarbons of 4 carbon atoms and hydrocarbons of 5 or more carbon atoms are shown in Table 3.

<Test Example 8A>

Lower hydrocarbons were synthesized from dimethyl ether and isobutene in substantially the same manner as in Test Example 3A, except that the reaction temperature was changed to 620°C.
The yields (weight %) of methane, hydrocarbons of 2 carbon atoms, propylene, propane, hydrocarbons of 4 carbon atoms and hydrocarbons of 5 or more carbon atoms are shown in Table 3.

[Table 3]

| | | Test Example 3A | Test Example 4A | Test Example 5A | Test Example 6A | Test Example 7A | Test Example 8A |
|---|---|---|---|---|---|---|---|
| Catalyst | | HCaMFI-B | HCaMFI-B | HCaMFI-B | HCaMFI-B | HCaMFI-B | HCaMFI-B |
| Temperature | [°C] | 380 | 430 | 480 | 530 | 580 | 620 |

(continued)

| | | Test Example 3A | Test Example 4A | Test Example 5A | Test Example 6A | Test Example 7A | Test Example 8A |
|---|---|---|---|---|---|---|---|
| Catalyst | | HCaMFI-B | HCaMFI-B | HCaMFI-B | HCaMFI-B | HCaMFI-B | HCaMFI-B |
| Feed flow rate [mmol/h] | Isobutene | 28. 6 | 28.6 | 28.6 | 28.6 | 28.6 | 28. 6 |
| | Nitrogen | 29. 0 | 29.0 | 29.0 | 29.0 | 29.0 | 29. 0 |
| | Total amount | 57. 6 | 57.6 | 57.6 | 57.6 | 57.6 | 57. 6 |
| Product distribution (weight %) | Methane | 0. 0 | 0.0 | 0.0 | 0.1 | 0.3 | 0. 8 |
| | C2 hydrocarbon | 0. 9 | 1.8 | 4.3 | 7.5 | 10.9 | 13. 2 |
| | Propylene | 11. 8 | 19.8 | 26.5 | 31.9 | 35.5 | 35. 9 |
| | Propane | 0. 8 | 1.0 | 1.6 | 1.7 | 1.6 | 1. 5 |
| | C4 hydrocarbon | 29. 2 | 33.7 | 33.5 | 32.4 | 32.7 | 33. 4 |
| | C5+ hydrocarbon | 57. 3 | 43.7 | 34.1 | 26.4 | 19.0 | 15. 2 |

Test Examples 3A to 8A were performed to observe the state of the decomposition reaction of olefin depending on the reaction temperature. From the results shown in Table 3, it was found that, as the reaction temperature is elevated, the amount of hydrocarbons of 5 or more carbon atoms tends to decrease whereas the amount of lower hydrocarbons increase.

FIG. 2 shows the yield (weight %) of methane indicated in Table 3 plotted against the reaction temperature (°C). From FIG. 2, it can be seen that the yield (weight %) of methane tends to increase as the reaction temperature becomes higher. Especially when the reaction temperature exceeds 600°C, the yield of methane increases rapidly. Therefore, it was found that the yield of methane which has no added value rapidly increases when the reaction temperature exceeds 600°C, and hence, it is highly possible that the economical efficiency of the process is lowered in such a case.

Example B

[Preparation of reaction catalyst]

A calcium-containing MFI zeolite was prepared in accordance with the preparation method described in Japanese Unexamined Patent Application, First Publication No. 2005-138000. Then, using hydrochloric acid, the prepared catalyst was converted into a proton type by a typical operation. The resulting catalyst was dried at 120°C for 5 hours, followed by calcination in air at 520°C for 10 hours, thereby obtaining a proton-type, calcium-containing MFI zeolite.

[Hydrogenation catalyst]

A Lindlar's catalyst, which is palladium having calcium carbonated supported thereon, can be used. For partial hydrogenation, a Lindlar's catalyst having Pb added thereto (5% Pd-Pb/$CaCO_3$) can be used to suppress side reaction which is hydrogenation of reaction of olefins.

[Synthesis of lower hydrocarbons]

Using the HCaMFI catalyst as a reaction catalyst, lower hydrocarbons were synthesized from hydrocarbons of 4 carbon atoms and/or hydrocarbons of 5 carbon atoms containing dimethyl ether and isobutene, hydrocarbons of 4 carbon atoms and/or hydrocarbons of 5 carbon atoms containing no dimethyl ether and isobutene, or only dimethyl ether.

<Test Example 1B>

Dimethyl ether, a mixed gas of hydrocarbons of 4 or 5 carbon atoms containing no butadiene, and nitrogen were mixed together at flow rates of 1,291 Ncm$^3$/hour, 645 Ncm$^3$/hour and 646 Ncm$^3$/hour, respectively. Then, the resulting mixture was transferred to an isothermal reactor filled with a HCaMFI catalyst, and reacted with the catalyst at 530°C under atmospheric pressure. The weight hourly space velocity (WHSV), which is the ratio of the supplied quantity of dimethyl ether (DME) as a raw material to the quantity of the catalyst, was set to be 9.6 g-DME/(g-catalyst · hour). The TG of the catalyst following 24 hours of reaction was measured, and the carbon production rate was determined from the TG.

<Test Example 2B>

Dimethyl ether, a mixed gas of hydrocarbons of 4 or 5 carbon atoms containing 5 % of butadiene, and nitrogen were mixed together at flow rates of 1,291 Ncm$^3$/hour, 645 Ncm$^3$/hour and 646 Ncm$^3$/hour, respectively. Then, the resulting mixture was transferred to an isothermal reactor filled with a HCaMFI catalyst, and reacted with the catalyst at 530°C under atmospheric pressure. The weight hourly space velocity (WHSV), which is the ratio of the supplied quantity of dimethyl ether (DME) as a raw material to the quantity of the catalyst, was set to be 9.6 g-DME/(g-catalyst · hour). The TG of the catalyst following 24 hours of reaction was measured, and the carbon production rate was determined from the TG.

<Comparative Example 1B>

Dimethyl ether, a mixed gas of hydrocarbons of 4 or 5 carbon atoms containing 40 % of butadiene, and nitrogen were mixed together at flow rates of 1,291 Ncm$^3$/hour, 645 Ncm$^3$/hour and 646 Ncm$^3$/hour, respectively. Then, the resulting mixture was transferred to an isothermal reactor filled with a HCaMFI catalyst, and reacted with the catalyst at 530°C under atmospheric pressure. The weight hourly space velocity (WHSV), which is the ratio of the supplied quantity of dimethyl ether (DME) as a raw material to the quantity of the catalyst, was set to be 9.6 g-DME/(g-catalyst · hour). The TG of the catalyst following 24 hours of reaction was measured, and the carbon production rate was determined from the TG.

<Comparative Example 2B>

Dimethyl ether and nitrogen were mixed together at flow rates of 1,291 and 1,291 Ncm$^3$/hour, respectively. Then, the resulting mixture was transferred to an isothermal reactor filled with the HCaMFI catalyst, and reacted with the catalyst at 530°C under atmospheric pressure. The weight hourly space velocity (WHSV), which is the ratio of the supplied quantity of dimethyl ether (DME) as a raw material to the quantity of the catalyst, was set to be 9.6 g-DME/(g-catalyst · hour). The TG of the catalyst following 24 hours of reaction was measured, and the carbon production rate was determined from the TG.
The results of Examples 1B and 2B, and Comparative Examples 1B and 2B are shown in Table 4.
[Table 4]

| | | | Example 1B | Example 2B | Comparative Example 1B | Comparative Example 2B |
|---|---|---|---|---|---|---|
| Feed flow rate of raw materials (Ncm$^3$/hour) | | DME | 1291 | 1291 | 1291 | 1291 |
| | C4/C5 mixed gases (Butadiene conter | | 645 | 645 | 645 | 0 |
| | | | 0% | 5% | 40% | 0% |
| | Nitrogen | | 646 | 646 | 646 | 1291 |
| Carbon production rate (mg-Carbon/ (g-catalyst hour)) | | | 3. 3 | 5. 5 | 8. 2 | 7. 0 |

As shown in Table 4, from the results of Examples 1B and 2B and Comparative Examples 1B and 2B, the production rate of carbon in Comparative Example 1B in which hydrocarbons of 4 carbon atoms and/or 5 carbon atoms having a butadiene content of 40% were mixed in the raw gaseous mixture was higher than that in Comparative Example 2B in which no hydrocarbons of 4 carbon atoms and/or 5 carbon atoms were contained in the raw gaseous mixture. Therefore, it was found that, when hydrocarbons of 4 carbon atoms and/or hydrocarbons of 5 carbon atoms having a high butadiene content was supplied to the reactor with at least one member selected from the group consisting of dimethyl ether and methanol, the influence on the deactivation of the catalyst is large as compared to when hydrocarbons of 4 carbon atoms and/or hydrocarbons of 5 carbon atoms are not supplied to the reactor.

Further, from a comparison of Comparative Example 1B with Examples 1B and 2B, it was found that the production rate of carbon in Example 2B in which the butadiene content was as low as 5% was lower than that in Comparative Example 1B in which the butadiene content was as high as 40%, and the production rate of carbon in Example 1B in which no butadiene was contained decreased significantly as compared to the production rate of carbon in Comparative Example 1B. Therefore, it was found that, even when hydrocarbons of 4 carbon atoms and/or hydrocarbons of 5 carbon atoms were supplied to the reactor, the production rate of carbon can be decreased by partial hydrogenation of butadiene to reduce the content of dienes. As a result, the load on the catalyst could be reduced significantly.

Furthermore, in Examples 1B and 2B in which the butadiene content was low, the reaction test could be performed without causing any problems. On the other hand, in Comparative Example 1B in which the butadiene content was high, carbon substances were deposited in preheated pipes (about 400°C), and hence, ΔP increased. As a result, problems were caused by clogging of pipes.

From the above, it was found that by partially hydrogenating dienes such as butadiene contained in hydrocarbons of 4 carbon atoms and/or hydrocarbons of 5 carbon atoms to convert the dienes into olefins, the production rate of carbon can be decreased, and hence, deposition of solid fractions within the pipes can be effectively prevented.

Further, it was also found that the durability of the reaction catalyst filled in the reactor can be enhanced.

**Claims**

1. A method for producing propylene comprising:

   transferring a feed gas into a reactor, said feed gas comprising at least one member selected from the group consisting of dimethyl ether and methanol and at least one member selected from the group consisting of olefins of 4 carbon atoms and olefins of 5 carbon atoms; and
   reacting said feed gas in the presence of a catalyst,
   the feed gas prior to transferring into the reactor having a ratio of the total supplied quantity of olefins of 4 carbon atoms and olefins of 5 carbon atoms to the total supplied quantity of dimethyl ether and methanol supplied within the range from 0.25 to 7.5, in terms of the molar ratio based on the number of carbon atoms, and
   said feed gas being contacted with said catalyst at a temperature of 350°C to 600°C.

2. The method according to Claim 1, wherein said at least one member selected from the group consisting of olefins of 4 carbon atoms and olefins of 5 carbon atoms comprises a product obtained by producing an olefin using an olefin production device and subjecting the olefin to separation using a separator.

3. The method according to Claim 1, wherein said at least one member selected from the group consisting of olefins of 4 carbon atoms and olefins of 5 carbon atoms is a product obtained by subjecting an olefin produced using an olefin production device and the reaction product obtained in said reactor to separation using a separator.

4. The method according to Claim 2, wherein the reaction product obtained in said reactor contains propylene as a main component, and the reaction product is supplied back to said separator to separate propylene from other components.

5. The method according to Claim 2 or 3, wherein said olefin production device is a thermal-cracking device for hydrocarbons and/or a catalytic-cracking device for hydrocarbons.

6. The method according to Claim 1, wherein said catalyst is a MFI zeolite catalyst.

7. The method according to Claim 1, wherein said catalyst comprises a MFI zeolite catalyst containing an alkaline earth metal, and
   said MFI zeolite catalyst having a Si/Al molar ratio of 10 to 300, and an alkaline earth metal/Al molar ratio of 0.75 to 15.

8. A method for producing propylene comprising:

   transferring a feed gas into a reactor, said feed gas comprising at least one member selected from the group consisting of dimethyl ether and methanol and hydrocarbons of 4 carbon atoms and/or hydrocarbons of 5 carbon atoms; and

reacting said feed gas in the presence of a reaction catalyst,
said hydrocarbons of 4 carbon atoms and/or hydrocarbons of 5 carbon atoms comprising at least one member selected from the group consisting of alkynes and dienes, and
said hydrocarbons of 4 carbon atoms and/or hydrocarbons of 5 carbon atoms having hydrogen supplied thereto to convert said at least one member selected from the group consisting of alkynes and dienes to an olefin having one double bond, and the olefin having one double bond being supplied into said reactor with said at least one member selected from the group consisting of dimethyl ether and methanol.

9.  The method according to Claim 8, wherein said hydrocarbons of 4 carbon atoms and/or hydrocarbons of 5 carbon atoms comprises a product obtained by producing an olefin using an olefin production device and subjecting the olefin to separation using a separator.

10. The method according to Claim 9, wherein said olefin production device is at least one member selected from the group consisting of a thermal-cracking device for hydrocarbons, a catalytic-cracking device for hydrocarbons and a device for subjecting an oxygen-containing hydrocarbon to dehydration-condensation reaction.

11. The method according to Claim 8, wherein at least a part of the hydrogen to be supplied to said hydrocarbons of 4 carbon atoms and/or hydrocarbons of 5 carbon atoms is produced in said reactor.

12. The method according to Claim 8, wherein said catalyst is a MFI zeolite catalyst.

13. The method according to Claim 8, wherein said catalyst comprises a MFI zeolite catalyst containing an alkaline earth metal, and
said MFI zeolite catalyst has a Si/Al molar ratio of 10 to 300, and an alkaline earth metal/Al molar ratio of 0.75 to 15.

14. An apparatus for producing propylene comprising:

a hydrogenation reactor in which hydrogen is supplied to hydrocarbons of 4 carbon atoms and/or hydrocarbons of 5 carbon atoms containing at least one member selected from the group consisting of alkynes and dienes , and said at least one member selected from the group consisting of alkynes and dienes is partially hydrogenated to be converted into an olefin having one double bond;
a reactor in which the resulting hydrocarbons of 4 carbon atoms and/or hydrocarbons of 5 carbon atoms obtained in said hydrogenation reactor is reacted with at least one member selected from the group consisting of dimethyl ether and methanol in the presence of a catalyst; and
a separator for separating propylene from the reaction product obtained in said reactor.

15. The apparatus according to Claim 14, wherein said hydrogenation reactor consist of hydrogenation catalyst containing palladium.

16. The apparatus according to Claim 14 or 15, wherein at least a part of the hydrogen to be supplied to said feed gas is produced in said reactor.

# FIG. 1

# FIG. 2

# FIG. 3

RAW MATERIALS
↓ 21

OLEFIN PRODUCTION DEVICE — 11

10

HYDROCARBONS OF 4 CARBON ATOM/
HYDROCARBONS OF 5 CARBON ATOMS

23

HYDROGEN →

HYDROCARBONS OF 4 CARBON ATOM/
HYDROCARBONS OF 5 CARBON ATOMS

24

22

12 — HYDROGENATION REACTOR

REACTOR

SEPARATOR → GASOLINE
→ PROPYLENE
→ WATER

DIMETHYL ETHER/METHANOL

25

26

13

14

EP 2 058 290 A1

## INTERNATIONAL SEARCH REPORT

| International application No. |
| --- |
| PCT/JP2007/066395 |

A. CLASSIFICATION OF SUBJECT MATTER
*C07C1/24*(2006.01)i, *B01J29/40*(2006.01)i, *C07C4/02*(2006.01)i, *C07C4/08* (2006.01)i, *C07C11/06*(2006.01)i

According to International Patent Classification (IPC) or to both national classification and IPC

B. FIELDS SEARCHED

Minimum documentation searched (classification system followed by classification symbols)
C07C1/24, B01J29/40, C07C4/02, C07C4/08, C07C11/06

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)
CAplus(STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | MARTIN, A. et al., Coupled conversion of methanol and C4 hydrocarbons to lower olefins, Applied Catalysis, 50(2), 1989, p.149-155 (particularly, pages 153 to 154) | 1-5<br>8-11,14-16<br>6,7,12,13 |
| X<br>Y | US 2003/0181777 A1 (LYONDELL CHEMICAL CO.), 25 September, 2003 (25.09.03), Par. Nos. [0023] to [0030] (Family: none) | 1-7<br>8-16 |
| X<br>Y<br>A | JP 61-24526 A (Director General, Agency of Industrial Science and Technology), 03 February, 1986 (03.02.86), Claims; page 5, upper left column, line 4 to page 7 & DE 3524890 A1 | 1-5<br>8-11,14-16<br>6,7,12,13 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>12 November, 2007 (12.11.07) | Date of mailing of the international search report<br>27 November, 2007 (27.11.07) |
| --- | --- |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2007/066395 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>Y<br>A | JP 62-70325 A (Director General, Agency of Industrial Science and Technology), 31 March, 1987 (31.03.87), Claims; page 4, upper left column, line 2 to page 5; Fig. 1 (Family: none) | 1-5<br>8-11,14-16<br>6,7,12,13 |
| Y<br>A | WO 2006/009099 A1 (Asahi Kasei Chemicals Corp.), 26 January, 2006 (26.01.06), Par. Nos. [0014], [0015] (Family: none) | 8-16<br>1-7 |
| A | WO 2005/016856 A1 (TOTALPETROCHEMICALS RESEARCH FELUY), 24 February, 2005 (24.02.05), Claims; pages 21 to 29 & EP 1508555 A1 & US 2007/027351 A1 | 1-16 |
| A | NOWAK, S. et al., An improved method for producing low olefins and gasoline by coupled methanol/hydrocarbon cracking (CMHC), Proc. – Int. Congr. Catal, 4, 1988, p.1735-1742 | 1-16 |
| P,X<br>P,A | JP 2007-51143 A (China Petroleum and Chemical Corp.), 01 March, 2007 (01.03.07), Claims; Par. Nos. [0059] to [0079] & US 2007/038010 A1 | 1-5<br>6-16 |
| P,X<br>P,A | CN 1915937 A (China Petroleum and Chemical Corp.), 21 February, 2007 (21.02.07), Example 1 (Family: none) | 1<br>2-16 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006234007 A
- JP 2006264513 A
- JP 62179592 A
- JP 60120790 A
- JP 2005138000 A